# EUROPÄISCHE PATENTSCHRIFT

(11) **EP 0 934 733 B1**
(45) Veröffentlichungstag und Bekanntmachung des Hinweises auf die Patenterteilung: **17.09.2003**
(21) Anmeldenummer: 98102224.7
(22) Anmeldetag: 09.02.1998
(51) Int. Cl.: A61F 9/007

(54) **Vorrichtung zum Schutz von Augenorganen, welche im Bereich der Vorderkammer eines Auges liegen, bei einer Operation an der Augenlinse**
Device for protection of eye parts lying in the anterior chamber of an eye during lens operation
Dispositif de protection pour parties de l'oeil dans la région anterieure d'un oeil pendant une opération de lentille

(43) Veröffentlichungstag der Anmeldung: 11.08.1999
(73) Patentinhaber: Tomalla, Karin, 45472 Mülheim/Ruhr (DE)
(72) Erfinder: Tomalla, Mark, Dr., 45472 Mülheim/Ruhr (DE)
(74) Vertreter: Nöth, Heinz, Dipl.-Phys.

(56) Entgegenhaltungen:
- EP-A- 0 630 611
- WO-A-93/14702
- US-A- 5 071 421
- US-A- 5 320 113
- US-A- 5 374 272

## Beschreibung

Die Erfindung betrifft eine Vorrichtung zum Schutz von Augenorganen nach dem Oberbegriff des Patentanspruches 1.

Bei Operationen an der Augenlinse, insbesondere Kataraktoperationen, ergeben sich bei bestimmten Augentypen Komplikationen in Form eines Prolapses der Iris oder Pigmentverlust im Irisgewebe, wenn das bei der Operation zum Einsatz kommende Werkzeug, beispielsweise eine Phakosonde, mit dem Gewebe in Kontakt kommt. Diese Schwierigkeiten treten vor allem bei engen Pupillen, z.B. nach Miotikatherapie oder auch bei hyperopen Augen mit temporär flacher Vorderkammer auf. Auch bei intraoperativem Druck von hinten, wie er bei lokaler A-Anästhesie von Asthmatikern oder adipösen Patienten auftreten kann, können die oben genannten Schwierigkeiten vorkommen.

Aus der US-A-5,374,272, von welcher im Oberbegriff des Patentanspruches 1 ausgegangen wird, ist es zum Schutz der Augeniris bekannt, einen Ring mit einem Halteteil und Einschubteil auf den Innenrand der Iris aufzusetzen. Diese ringförmige Schutzvorrichtung wird von einem Folienmaterial aus nichttoxischem und wärmebeständigen Kunststoff gebildet. Der Ring besitzt in einem innenliegenden Bereich eine geringere Breite als in einem äußeren Bereich.

Aufgabe der Erfindung ist es daher, eine Vorrichtung der eingangs genannten Art zu schaffen, welche während der Ope-Operation an der Augenlinse, insbesondere bei einer Katarakt-Operation, zum Schutz der im Bereich der Vorderkammer eines Auges liegenden Augenorganen einfach handhabbar ist.

Diese Aufgabe wird erfindungsgemäß durch die Schaffung einer Vorrichtung der eingangs genannten Art gelöst, welche die kennzeichnenden Merkmale des Patentanspruches 1 aufweist.

Hierzu wird ein Einschubteil vorgesehen, welches mit einer die Iris zumindest teilweise insbesondere jedoch im wesentlichen abdeckenden Einschublänge durch den Tunnel in die Vorderkammer des Auges einschiebbar ist. Dieses Einschubteil ist mit einem außerhalb des Tunnels an der Außenfläche des Auges im Bereich der Sklera bzw. Lederhaut . abstützbaren Halteteil verbunden. Das Halteteil besitzt in bevorzugter Weise Stützflächen, die sich seitlich vom äußeren Tunnelende an der Außenfläche der Hornhaut bzw. Lederhaut abstützen können. Das Einschubteil und Halteteil sind einstückig aus einer flexiblen Folie, insbesondere durch Stanzen aus einer Folienbahn, gebildet. Die beim Stanzen entstandenen Kanten werden insbesondere durch Polieren abgerundet. In dem Bereich, welcher bei der Operation im Tunnel liegt, besitzt das Einschubteil eine geringere Breite als am vorderen, etwa 3mm in die Vorderkammer ragenden Ende. Durch die Breitenverringerung wird gewährleistet, daß während der Operation Spül- bzw. Kühlflüssigkeit, welche insbesondere bei der Phakoemulsifikation gebraucht wird, seitlich durch den Tunnel fließen und austreten kann. Durch das verbreiterte vordere Ende des Einschubteils wird eine möglichst große Schutzfläche erzielt, welche bei der Operation, insbesondere bei der Phakoemulsifikation im Rahmen einer Kataraktoperation, die unterhalb des Einschubteils liegende Iris gegen Berührung mit einer an der Oberseite des Einschubteils in die Vorderkammer geführten Operationswerkzeug, beispielsweise einer Phakosonde, schützt. Hierdurch wird die Führung des Operationswerkzeugs, insbesondere der Phakosondenspitze, zum Behandlungsort in die Hinterkammer ohne Berührung der Iris erleichtert. Da das Einschubteil an seinem rückwärtigen Ende mit dem Halteteil verbunden ist, das an der Außenseite des Auges abgestützt ist, wird eine Fixierung des Einschubteils während des operativen Eingriffs erreicht. Durch das in die Vorderkammer eingeführte Einschubteil wird ferner ein Prolaps des Irisgewebes durch die Tunnelöffnung verhindert.

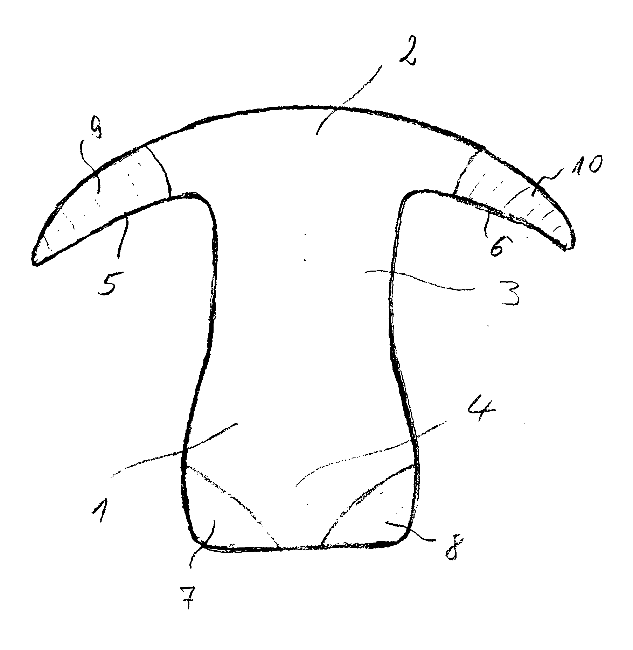

Anhand der Figur wird an einem Ausführungsbeispiel die Erfindung noch näher erläutert. Die Figur zeigt eine Draufsicht auf die Schutzvorrichtung, welche ein Einschubteil 1 aufweist, das durch einen insbesondere in der Cornea, z.B. im Limbusbereich, angelegten Tunnel einschiebbar ist. Die Länge des Einschubteils 1 ist so bemessen, daß im wesentlichen die Iris in radialer Richtung abgedeckt wird. Am hinteren Ende ist das Einschubteil 1 mit einem Halteteil 2 verbunden. Mit Hilfe einer beispielsweise am Halteteil 2 angreifenden Pinzette kann das Einschubteil 1 durch den Tunnel in die Vorderkammer eingesetzt werden, nachdem die Vorderkammer mit einem Viskoelastikum gestellt wurde. Zur besseren Gleitfähigkeit des Operationswerkzeugs, beispielsweise der Phakosondenspitze, kann auch die Oberfläche des Einschubteils 1 mit einem Viskoelastikum versehen werden. Im eingesetzten Zustand befindet sich der vordere Teil 4 des Einschubteils etwa 3 mm in der Vorderkammer des Auges und überdeckt im wesentlichen in radialer Richtung die Iris des Auges. Die Breite dieses vorderen Teiles ist so bemessen, daß die Bewegungen des Operationswerkzeuges, beispielsweise der Phakosondenspitze, auf der Oberfläche des Einschubteiles 1 erfolgen. Das verbreiterte vordere Ende 4 des Einschubteils 1 kann beispielsweise eine Breite von 3,5 mm haben und etwa der Schnittbreite des Tunnels entsprechen. Ein Bereich 3 des Einschubteiles 1, welcher sich während der Operation im Hornhauttunnel befindet, besitzt eine geringere Breite als der Bereich 4. Diese Breite kann etwa 3,2 mm betragen. Der verjüngte Bereich 3 gewährleistet, daß Spülflüssigkeit bzw. Kühlflüssigkeit, welche insbesondere bei der Phakoemulsifikation zum Einsatz kommt, durch den Tunnel ungehindert austreten kann. Der verjüngte Bereich 3 des Einschubteiles 1 besitzt eine geringere Breite als der Tunnel. Hierdurch wird Verbrennungen an der Cornea vorgebeugt.

Das Halteteil 2 am hinteren Ende des Einschubteiles 1 ist wesentlich breiter ausgebildet als die Schnittöffnung am Tunnelende. Das Halteteil 2 besitzt Stützkanten bzw. Stützflächen 5 und 6, welche seitlich vom äußeren Tunnelende an der Außenfläche an der Hornhaut bzw. am Limbus anliegen. Die Gestalt der Schutzvorrichtung gemäß dem Ausführungsbeispiel ist in der Draufsicht pilzförmig. Während der Operation übt die Vorrichtung eine Stabilisatorwirkung aus, da insbesondere das Irisgewebe im wesentlichen in seiner natürlichen Position gehalten werden kann.

Das Entfernen der Vorrichtung aus dem Auge kann mit Hilfe einer Pinzette, die am auβen liegenden Halteteil 2 angreift, herausgezogen werden. Damit während der Operation an der Hornhautaußenseite über die Stützflächen 5 und 6 eine sichere Abstützung gewährleistet wird, ist das Halteteil 2 wesentlich breiter ausgebildet als die Tunnelöffnung und kann beispielsweise 4,2 mm beim Ausführungsbeispiel betragen.

Die Schutzvorrichtung ist einstückig aus einem Folienmaterial gebildet und besitzt in bevorzugter Weise eine Dicke in der Größenordnung von 50 bis 100 µm. Als Folienmaterial eignen sich Polyester, beispielsweise Polyethylenterephtalat (PET), Polyesterkautschuke oder Polytetrafluorethylen und andere temperaturbeständige nichttoxische Kunststoffe. Der Körper der pilzförmigen Schutzfolie kann durch Stanzen aus einer Folienbahn gewonnen werden, wobei die Stanzkanten durch Polieren abgerundet werden.

An den beiden seitlichen etwa dreieckförmig gebildeten vorderen Randbereichen 7 und 8 des Einschubteils 1 kann das Folienmaterial eine höhere Steifigkeit bzw. Festigkeit aufweisen als im übrigen Bereich des Folienkörpers. In gleicher Weise können im Bereich der Stützflächen 5 und 6 die beiden seitlichen Enden 9 und 10 des Halteteils 2 eine höhere Steifigkeit bzw. Festigkeit aufweisen als das übrige Folienmaterial. Die Bereiche 7 bis 10 des Folienkörpers können etwa die gleiche Steifigkeit bzw. Festigkeit aufweisen

Insbesondere während der Phakoemusifikation wird durch die dargestellte stabilisierend wirkende Schutzfolie das Irisgewebe auch gegenüber thermischer Belastung durch die Phakosondenspitze geschützt. Die Schutzfolie bietet ein einfach handhabbares Hilfsmittel bei der Durchführung von operativen Eingriffen im Bereich der Vorder- und Hinterkammer des Auges, insbesondere beim Entfernen der natürlichen Augenlinse durch Phakoemulsifikation.

## Patentansprüche

1. Vorrichtung zum Schutz von Augenorganen mit einem Ein-Einschubteil (1), welches mit einem Halteteil (2) verbunden ist, wobei das Einschubteil (1) einen Bereich (3) geringerer Breite aufweist als in einem anderen Bereich (4) und das Einschubteil (1) und das Halteteil (2) einstückig aus einer flexiblen Folie gebildet sind, worin
der breitere Bereich (4) des Einschubteils (1) am vorderen Ende des Einschubteils (1) vorgesehen ist, das Halteteil mit dem die geringere Breite aufweisenden Bereich (3) des Einschubteils (1) verbunden ist und dass das Halteteil (2) seitlich vom Einschubteil (1) abstehende Stützflächen (5, 6) aufweist, **dadurch gekennzeichnet, dass** das Einschubteil (1) und das Halteteil (2) als in Draufsicht pilzförmige Folie ausgebildet sind und das Halteteil (2) im Bereich der Stützflächen (5, 6) eine gegenüber dem übrigen Bereich des Halteteils (2) erhöhte Steifigkeit oder Festigkeit aufweist.

2. Vorrichtung nach Anspruch 1,
**dadurch gekennzeichnet, dass** vordere Randbereiche (7, 8) des Einschubteils (1) eine höhere Steifigkeit aufweisen als der übrige Bereich des Einschubteils.

3. Vorrichtung nach einem der Ansprüche 1 bis 2,
**dadurch gekennzeichnet, dass** das Folienmaterial, aus welchem das Einschubteil (1) und das Halteteil (2) einstückig gebildet sind, aus einem nichttoxischen Kunststoff besteht.

4. Vorrichtung nach einem der Ansprüche 1 bis 3,
**dadurch gekennzeichnet, dass** das Folienmaterial aus einem wärmebeständigen Kunststoff besteht.

## Claims

1. A device for the protection of organs of the eye comprising an insertion portion (1) which is connected to a holding portion (2), wherein the insertion portion (1) has a region (3) of smaller width than in another region (4) and the insertion portion (1) and the holding portion (2) are integrally formed from a flexible film, wherein the wider region (4) of the insertion portion (1) is provided at the front end of the insertion portion (1), the holding portion is connected to the region (3) of the insertion portion (1), which is of the smaller width, and the holding portion (2) has support surfaces (5, 6) projecting laterally from the insertion portion (1), **characterised in that** the insertion portion (1) and the holding portion (2) are in the form of a film which is mushroom-shaped in plan view and the holding portion (2) in the region of the support surfaces (5, 6) is of a stiffness or strength which is increased in comparison with the other region of the holding portion (2).

2. A device according to claim 1 **characterised in that** front edge regions (7, 8) of the insertion portion (1) have a higher degree of stiffness than the other region of the insertion portion.

3. A device according to one of claims 1 and 2 **characterised in that** the film material from which the insertion portion (1) and the holding portion (2) are integrally formed comprises a non-toxic plastic material.

4. A device according to one of claims 1 to 3 **characterised in that** the film material comprises a heat-resistant plastic material.

## Revendications

1. Dispositif de protection pour parties de l'oeil comprenant une partie insérable (1), qui est assemblée avec une partie de maintien (2), la partie insérable (1) présentant une zone (3) de largeur plus faible que dans une autre zone (4), et la partie insérable (1) et la partie de maintien (2) étant formées d'une seule pièce d'une feuille flexible, dispositif dans lequel la zone plus large (4) de la partie insérable (1) est prévue sur l'extrémités avant de la partie insérable (1), la partie de maintien est assemblée avec la zone (3) de largeur inférieure de la partie insérable (1), et la partie de maintien (2) présente des surfaces d'appui (5, 6) dépassant latéralement de la partie insérable (1), **caractérisé en ce que** la partie insérable (1) et la partie de maintien (2) sont configurées sous forme de feuille fongiforme en vue du dessus, et **en ce que** la partie de maintien (2) présente dans la zone des surfaces d'appui (5, 6) une rigidité ou une résistance accrue par rapport à l'autre zone de la partie de maintien (2).

2. Dispositif suivant la revendication 1, **caractérisé en ce que** des zones de bordure avant (7, 8) de la partie insérable (1) présentent une rigidité plus élevée que l'autre zone de la partie insérable.

3. Dispositif suivant l'une des revendications 1 et 2, **caractérisé en ce que** le matériau en feuille, dont sont formées d'une seule pièce la partie insérable (1) et la partie de maintien (2), se compose d'une matière plastique non toxique.

4. Dispositif suivant l'une des revendications 1 à 3, **caractérisé en ce que** le matériau en feuille se compose d'une matière plastique thermostable.
